(19) Europäisches Patentamt
European Patent Office
Office européen des brevets

(11) Veröffentlichungsnummer: **0 117 225 B1**

# (12) EUROPÄISCHE PATENTSCHRIFT

(45) Veröffentlichungstag der Patentschrift: **13.11.91**

(51) Int. Cl.[5]: **C07D 211/58**, C07D 211/26, C07D 211/28, C08K 5/34

(21) Anmeldenummer: **84810029.3**

(22) Anmeldetag: **18.01.84**

Die Akte enthält technische Angaben, die nach dem Eingang der Anmeldung eingereicht wurden und die nicht in dieser Patentschrift enthalten sind.

## (54) N-(Polyalkylpiperidinyl)-carbamate von Polyolen.

(30) Priorität: **24.01.83 CH 383/83**

(43) Veröffentlichungstag der Anmeldung: **29.08.84 Patentblatt 84/35**

(45) Bekanntmachung des Hinweises auf die Patenterteilung: **13.11.91 Patentblatt 91/46**

(84) Benannte Vertragsstaaten: **BE DE FR GB IT NL**

(56) Entgegenhaltungen:
**DE-A- 2 349 962**
**GB-A- 2 074 564**
**US-A- 3 937 711**
**FR-A- 2 074 871**
**US-A- 3 904 581**
**US-A- 4 191 683**

(73) Patentinhaber: **CIBA-GEIGY AG**
**Klybeckstrasse 141**
**CH-4002 Basel(CH)**

(72) Erfinder: **Karrer, Friedrich, Dr.**
**Rebbergstrasse 5**
**CH-4800 Zofingen(CH)**

**EP 0 117 225 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Derivate von Polyalkylpiperidinen, welche N-substituierte Carbamate von Di-, Tri- oder Tetraolen sind, sowie deren Verwendung als Stabilisatoren für Polymere, insbesondere gegen deren Schädigung durch Licht. Die Erfindung betrifft auch die mittels dieser neuen Verbindungen stabilisierten Polymeren.

US-A-3,937,711 beschreibt Piperidinyläthyl-amine wie z.B. 2(2',2',6',6'-Tetramethylpiperidinyl-4')-äthylamin, und ihre Verwendung als Lichtschutzmittel für Polyolefine.

In der DE-OS 2 040 975 werden Derivate von 4-Amino-2,2,6,6-tetramethylpiperidin als Lichtschutzmittel für Polymere beschrieben, darunter auch die entsprechenden 4-Alkoxy-carbonylamino-Derivate. Als Beispiel hierfür wird die Verbindung 4-($\beta$-Hydroxyethoxycarbonamido)-2,2,6,6,-tetramethylpiperidin (Verbindung Nr. 7) genannt und ihre Verwendung in verschiedenen Polymeren beschrieben.

In der DE-OS 2 349 962 werden ähnliche Verbindungen als Lichtschutzmittel empfohlen, die sich von den obigen Verbindungen im wesentlichen durch die Art der Substitution des Piperidin-Stickstoffes unterscheiden. Als Beispiel für ein 4-Carbamat ist dort die Verbindung 4-(2-Hydroxyethoxycarbonylamino)-1,2,2,6,6-pentamethylpiperidin (Verbindung Nr. 126) beschrieben sowie ihre Verwendung als Stabilisator in Polypropylen und Polyethylen hoher Dichte.

In der DE-OS 26 21 870 sind ebenfalls ähnliche Verbindungen als Lichtschutzmittel beschrieben, beispielsweise die Verbindung 4-Ethoxycarbonamido-2,6-diethyl-2,3,6-trimethylpiperidin.

Alle diese Verbindungen sind Monocarbamate mit einer Polyalkylpiperidin-Gruppe am Carbamat-Stickstoff. Sie sind ausgezeichnete Lichtschutzmittel, sind jedoch wegen ihres relativ niedrigen Molekulargewichtes für bestimmte Anwendungen nicht geeignet. Beispielsweise können sie aus dünnen Substratschichten rasch auswandern oder werden rasch ausgewaschen.

Polymere oder oligomere Carbamate (Polyurethane) mit Polyalkylpiperidingruppen, wie sie in der DE-OS 27 19 131 als Untergruppe 5 beschrieben sind, haben zwar ein relativ hohes Molekulargewicht, sodass sie nicht rasch durch Auswandern oder Auswaschen verlorengehen, jedoch ist ihre Lichtschutzwirkung in Polyolefinen wesentlich schwächer als die der Monocarbamate.

Es wurde nunmehr überraschend gefunden, dass Di-, Tri- und Tetracarbamate von Polyolen, deren Carbamat-Stickstoff durch Polyalkylpiperidingruppen substituiert ist, hervorragende Lichtschutzmittel für organische Materialien sind und dabei nicht die Nachteile der Monocarbamate oder der Polycarbamate des Standes der Technik aufweisen.

Gegenstand der Erfindung sind daher Verbindungen der Formel I,

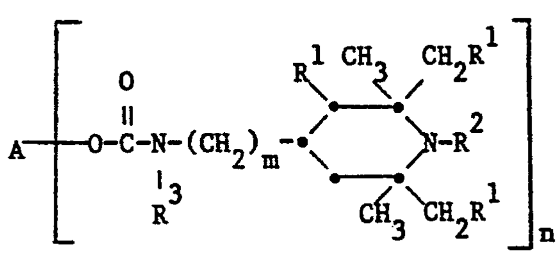

I

worin m null, 1 oder 2 und n 2, 3 oder 4 ist

- $R^1$ Wasserstoff oder $C_1$-$C_4$-Alkyl ist,
- $R^2$ Wasserstoff, $C_1$-$C_{12}$-Alkyl, $C_3$-$C_6$-Alkenyl, $C_3$-$C_5$-Alkinyl, $C_7$-$C_{12}$-Aralkyl, Glycidyl, Cyanomethyl oder eine der Gruppen -CO-NH-$R^4$, -CO-N($R^5$)$_2$, -COO$R^6$, -$CH_2$COO$R^6$, -$CH_2$CON($R^5$)$_2$, -CO-$R^7$ oder -$CH_2$CH($R^8$)O$R^9$ bedeutet,
- $R^3$ Wasserstoff, $C_1$-$C_{20}$-Alkyl, durch -O$R^{10}$, -OOC-$R^{11}$, -CN, -COO$R^{12}$, -N($R^{13}$)($R^{14}$) oder -CON($R^{13}$)-($R^{14}$) substituiertes $C_1$-$C_6$-Alkyl, $C_3$-$C_5$-Alkenyl, $C_5$-$C_8$-Cycloalkyl, $C_6$-$C_{12}$-Cycloalkyl-alkyl, $C_6$-$C_{10}$-Aryl, $C_7$-$C_{15}$-Alkylaryl, $C_7$-$C_{12}$-Aralkyl oder eine Gruppe der Formel II bedeutet,

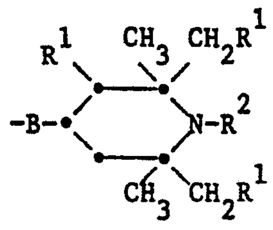

II

worin B eine direkte Bindung, $-CH_2CH_2-$ oder $-CH_2CH_2O-$ bedeutet,

$R^4$ $C_1$-$C_8$-Alkyl, $C_5$-$C_8$-Cycloalkyl, $C_6$-$C_{10}$-Aryl oder $C_7$-$C_{15}$-Alkylaryl bedeutet,

$R^5$ $C_1$-$C_8$-Alkyl, Cycloalkyl, Phenyl, Benzyl ist oder beide Reste $R^5$ zusammen mit dem N-Atom, an das sie gebunden sind, einen 5- bis 7-gliedrigen heterocyclischen Ring bilden,

$R^6$ $C_1$-$C_{12}$-Alkyl, $C_5$-$C_8$-Cycloalkyl, Allyl, Benzyl oder Phenyl bedeutet,

$R^7$ Wasserstoff, $C_1$-$C_{11}$-Alkyl, $C_2$-$C_5$-Alkenyl, $C_5$-$C_8$-Cycloalkyl, Phenyl oder $C_7$-$C_{12}$-Aralkyl bedeutet,

$R^8$ Wasserstoff, Methyl, Ethyl, Phenyl, Butoxymethyl oder Phenoxymethyl bedeutet,

$R^9$ Wasserstoff, $C_1$-$C_8$-Alkyl, $C_3$-$C_6$-Alkenyl, Benzyl oder $-CO-R^{11}$ bedeutet,

$R^{10}$ Wasserstoff, $C_1$-$C_{12}$-Alkyl oder $C_5$-$C_8$-Cycloalkyl bedeutet,

$R^{11}$ $C_1$-$C_{11}$-Alkyl, $C_2$-$C_5$-Alkenyl, $C_5$-$C_8$-Cycloalkyl, Phenyl, $C_7$-$C_{12}$-Aralkyl, durch $C_1$-$C_4$-Alkyl und/oder OH substituiertes Phenyl oder $C_7$-$C_9$-Phenylalkyl oder eine Gruppe $-NH-R^4$ bedeutet,

$R^{12}$ $C_1$-$C_{12}$-Alkyl, $C_5$-$C_8$-Cycloalkyl, Allyl, Benzyl, durch OH und $C_1$-$C_4$-Alkyl im Phenylrest substituiertes $C_7$-$C_9$-Phenylalkyl, Phenyl oder eine Gruppe der Formel II bedeutet,

$R^{13}$ $C_1$-$C_8$-Alkyl, Cyclohexyl, Benzyl, Phenyl oder einen Rest der Formel II, und

$R^{14}$ Wasserstoff, $C_1$-$C_8$-Alkyl, Cyclohexyl oder einen Rest der Formel II bedeutet oder $R^{14}$ zusammen mit $R^{13}$ und dem N-Atom einen 5-7-gliedrigen heterocyclischen Rest bildet,

A der n-wertige Rest eines Polyols ist, und wenn n 2 ist, $C_2$-$C_{16}$-Alkylen, durch ein oder mehrere -O- oder $-N(C_1$-$C_4$-Alkyl)- unterbrochenes $C_4$-$C_{20}$-Alkylen, $C_4$-$C_{10}$-Alkenylen, $C_6$-$C_8$-Cycloalkylen, $C_8$-$C_{12}$-Dialkylen-cycloalkan, $C_8$-$C_{14}$-Dialkylenaren, $C_6$-$C_{12}$-Arylen, $C_{13}$-$C_{18}$-Diphenylen-alkan oder eine Gruppe der Formel III bedeutet,

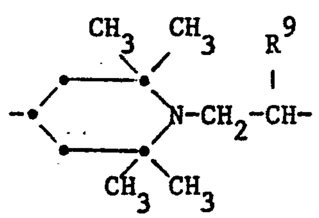

III

und wenn n 3 ist, A einen dreiwertigen aliphatischen oder cycloaliphatischen Kohlenwasserstoffrest mit 3-20 C-Atomen bedeutet, und wenn n 4 ist, einen vierwertigen aliphatischen Kohlenwasserstoffrest mit 4-20 C-Atomen bedeutet,
sowie Säure-Additonssalze dieser Verbindungen.

In dieser Formel kann $R^1$ ein $C_1$-$C_4$-Alkylrest sein, wie z.B. Methyl, Ethyl, Propyl, Isopropyl oder Butyl. $R^4$, $R^5$, $R^9$, $R^{13}$ und $R^{14}$ als $C_1$-$C_8$-Alkyl können dieselben Bedeutungen haben und können darüber hinaus auch z.B. Isoamyl, n-Hexyl, 2-Ethylbutyl, n-Heptyl, n-Octyl, 2-Ethylhexyl oder 1,1,3,3-Tetramethylbutyl sein. $R^7$ und $R^{11}$ als $C_1$-$C_{11}$-Alkyl können darüber hinaus auch z.B. Isononyl, n-Decyl oder n-Undecyl sein, $R^2$, $R^6$, $R^8$ und $R^{10}$ als $C_1$-$C_{12}$-Alkyl können darüber hinaus auch z.B. n-Dodecyl sein. $R^3$ als $C_1$-$C_{20}$-Alkyl kann darüber hinaus auch z.B. Tetradecyl, Hexadecyl, Octadecyl oder Eikosyl sein. Alle Alkylgruppen können unverzweigt oder verzweigt sein.

$R^3$, $R^4$, $R^6$, $R^7$, $R^{10}$, $R^{11}$ und $R^{12}$ können $C_5$-$C_8$-Cycloalkyl sein, wie z.B. Cyclopentyl, Cyclohexyl, Methylcyclohexyl, Cycloheptyl oder Cyclooctyl. $R^3$ als Cycloalkyl-alkyl kann z.B. Cyclopentylmethyl, Cyclohexylmethyl, 2-Cyclohexylethyl, 2-Cyclooctylethyl oder 3-Cyclohexylpropyl sein.

$R^2$, $R^3$ und $R^9$ als $C_3$-$C_6$-Alkenyl sind insbesondere ($C_2$-$C_5$-Alkenyl)-Alkyl wie z.B. Allyl, Methallyl, 3,3-Dimethylallyl oder 2,3,3-Trimethylallyl. $R^2$ als $C_3$-$C_5$-Alkinyl kann z.B. Propargyl oder 2-Butinyl sein.

$R^2$, $R^3$, $R^7$ und $R^{11}$ als $C_7$-$C_{12}$-Aralkyl sind insbesondere Phenylalkyl, wie z.B. Benzyl, 2-Phenylethyl, 3-Phenylpropyl oder 1-Phenylisopropyl.

$R^3$ und $R^4$ als $C_6$-$C_{10}$-Aryl können Phenyl oder Naphthyl sein. $R^3$ und $R^4$ als $C_7$-$C_{15}$-Alkylaryl sind

insbesondere Alkylphenyl, wie z.B. Tolyl, Xylyl, 4-tert.Butylphenyl, 4-Hexylphenyl oder 4-Octylphenyl.

$R^{11}$ als durch $C_1$-$C_4$-Alkyl und/oder OH substituiertes Phenyl oder $C_7$-$C_9$-Phenylalkyl kann z.B. Tolyl, Xylyl, 4-Isopropylphenyl, 2-Hydroxyphenyl, 3,5-Di-tert.butyl-4-hydroxyphenyl, 4-Methylbenzyl, 3-Hydroxybenzyl, 3-Methyl-4-hydroxy-5-tert.butylbenzyl oder 2-[3,5-Di-tert.butyl-4-hydroxyphenyl]-ethyl sein.

$R^{12}$ als durch OH und $C_1$-$C_4$-Alkyl im Phenylrest substituiertes $C_7$-$C_9$-Phenylalkyl kann z.B. 3,5-Di-tert.butyl-4-hydroxybenzyl oder 3-Methyl-4-hydroxy-5-tert.butylbenzyl sein.

Wenn $R^{13}$ und $R^{14}$ oder beide Reste $R^5$ zusammen mit dem N-Atom, an das sie gebunden sind, einen 5-7-gliedrigen heterocyclischen Ring bilden, so kann dies z.B. ein Pyrrolidin-, Piperidin-, Morpholin- oder Piperazinring sein. Ein solcher Piperazinring kann in 4-Stellung durch $C_1$-$C_4$-Alkyl substituiert sein.

Die Gruppe A als $C_2$-$C_{16}$-Alkylen kann unverzweigtes oder verzweigtes Alkylen sein, wie z.B. 1,2-Ethylen, 1,2-Propylen, 1,3-Propylen, 1,4-Butylen, 1,6-Hexylen, 1,8-Octylen, 2,2-Dimethyl-1,3-propylen, Dodecamethylen oder Hexadecamethylen.

A als durch -O- oder -N($C_1$-$C_4$-Alkyl)- unterbrochenes $C_4$-$C_{20}$-Alkylen kann z.B. 3-Oxa-1,5-pentylen, 4-Oxa-2,6-heptylen, 3,6-Dioxa-1,8-octylen, 3,6,9-Trioxa-1,11-undecylen, 3-Methylaza-1,5-pentylen, 4-Isopropylaza-2,6-heptylen oder 3-Butylaza-1,5-pentylen sein.

A als Cycloalkylen oder Dialkylen-cycloalkan kann z.B. 1,3- oder 1,4-Cyclohexylen, 1,4-Di(methylen)-cyclohexan oder Dicyclohexylmethan-4,4'-diyl sein.

A als $C_4$-$C_{10}$-Alkenylen kann z.B. 2-Butenylen-1,4, 3-Hexenylen-1,6 oder 2-Methyl-2-butenylen-1,4- sein.

A als $C_8$-$C_{14}$-Dialkylenaren kann z.B. 1,3- oder 1,4-Xylylen, 1,4-Dimethylennaphthalin oder 1,4-Diethylenbenzol sein. A als $C_6$-$C_{12}$-Arylen kann z.B. Phenylen, Naphthylen oder Diphenylen sein. A als $C_{13}$-$C_{18}$-Diphenylenalkan kann z.B. Di(p-phenylen)-methan oder Di(p-phenylen)-propan-2,2 sein.

A als dreiwertiger Kohlenwasserstoffrest mit 3-20 C-Atomen kann z.B. Propan-1,2,3-triyl, 1,1,1-Trimethylenethan, 1,1,1-Trimethylenpropan oder 1,2,4-Trimethylen-cyclohexan sein.

A als vierwertiger Kohlenwasserstoffrest mit 4-20 C-Atomen kann z.B. Butan-1,2,3,4-tetrayl oder Tetra-(methylen)-methan sein.

Bevorzugt sind Verbindungen der Formel I, worin $R^1$ Wasserstoff ist, Verbindungen der Formel I, worin n 2 ist, sowie Verbindungen der Formel I, worin $R^3$ nicht Wasserstoff ist.

Bevorzugt sind ferner solche Verbindungen der Formel I, worin m null und n 2 ist, $R^1$ Wasserstoff ist, $R^2$ Wasserstoff, $C_1$-$C_4$-Alkyl, Allyl, Benzyl, Cyanomethyl oder -CO-$R^7$ bedeutet, $R^3$ Wasserstoff, $C_1$-$C_{12}$-Alkyl, $C_2$-$C_3$-Hydroxyalkyl, Cyclohexyl, Phenyl, Benzyl, eine Gruppe der Formel II, $R^{11}$-$COOCH_2CH_2$-, $R^{12}$-OOC-$(CH_2)_p$-oder $R^{13}$-NH-CO-$(CH_2)_p$-bedeutet, worin p 1 oder 2 ist, $R^7$ $C_1$-$C_4$-Alkyl oder Vinyl ist, $R^{11}$ $C_1$-$C_4$-Alkyl, -NH-($C_1$-$C_4$-Alkyl),Phenyl, oder durch eine OH-Gruppe und zwei $C_1$-$C_4$-Alkylgruppen substituiertes Phenyl oder Phenylethyl bedeutet, $R^{12}$ $C_1$-$C_6$-Alkyl, Allyl, 3,5-Di-tert.butyl-4-hydroxybenzyl oder eine Gruppe der Formel II ist, $R^{13}$ $C_1$-$C_6$-Alkyl oder eine Gruppe der Formel II ist und A $C_2$-$C_{12}$-Alkylen, $C_4$-$C_6$-Mono-oder Dioxaalkylen oder eine Gruppe der Formel III bedeutet.

Unter diesen Verbindungen sind insbesondere solche bevorzugt, worin $R^3$ $C_1$-$C_{12}$-Alkyl, $C_2$-$C_3$-Hydroxyalkyl, Cyclohexyl, eine Gruppe der Formel II oder der Formel $R^{12}$-OOC-$(CH_2)_p$- ist, worin p 1 oder 2 ist und $R^{12}$ $C_1$-$C_4$-Alkyl oder eine Gruppe der Formel II ist, sowie solche Verbindungen, worin A $C_2$-$C_6$-Alkylen oder 3-Oxa-1,5-pentylen bedeutet.

Sofern die Verbindungen der Formel I basische Verbindungen sind, können sie Säure-Additionssalze mit anorganischen oder organischen Säuren bilden. Beispiele für solche Säuren sind Phosphorsäure, Phenylphosphorsäure, Diethylphosphorsäure, Essigsäure, Ameisensäure, Toluolsulfonsäure, Methansulfonsäure, Phenylphosphonsäure oder Diphenylphosphinsäure.

Beispiele für einzelne Verbindungen der Formel I, worin n 2 ist, sind die Verbindungen der folgenden Formeln:

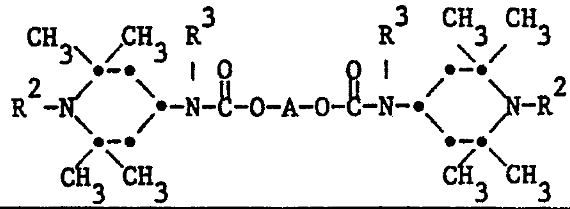

| $R^2$ | $R^3$ | A |
|---|---|---|
| H | H | $-(CH_2)_6-$ |
| H | $CH_3$ | $-(CH_2)_6-$ |
| $CH_3$ | $n-C_4H_9$ | $-(CH_2)_6-$ |
| H | $n-C_4H_9$ | $-(CH_2)_4-$ |
| $CH_3CO-$ | $n-C_3H_7$ | $-(CH_2)_8-$ |
| H | $n-C_8H_{17}$ | 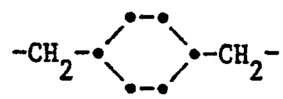 |
| $CH_3CO-$ | $n-C_4H_9$ | $-CH_2CH_2OCH_2CH_2-$ |
| H | $n-C_4H_9$ | $-CH_2CH_2N(CH_3)CH_2CH_2-$ |
| $CH_2=CHCO-$ | $n-C_4H_9$ | $-CH_2CH=CHCH_2-$ |
| $CH_3$ | $n-C_{12}H_{25}$ | 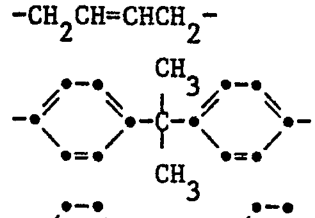 |
| $CH_2=CH_2CH_2-$ | H | 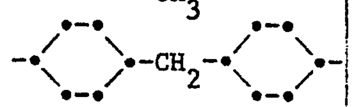 |
| $NC-CH_2-$ | 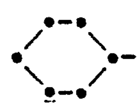 | $-(CH_2)_4-$ |
| 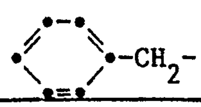 | 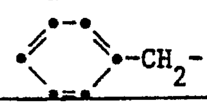 | $-(CH_2)_{12}-$ |

| $R^2$ | $R^3$ | A |
|---|---|---|
| $CH_3$ | 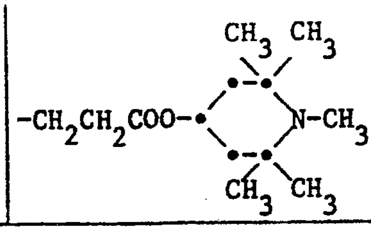 | $-(CH_2)_6-$ |

Beispiele für Verbindungen der Formel I, worin n 3 ist, sind die Verbindungen der folgenden Formeln

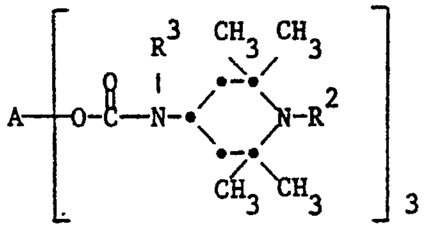

| $R^2$ | $R^3$ | A |
|---|---|---|
| H | $n-C_4H_9$ | $-CH_2-\overset{\vert}{C}H-CH_2-$ |
| H | $iso-C_8H_{17}$ | 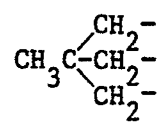 |
| $CH_3$ | $-CH_2CH_2COOC_2H_5$ | $CH_3C(CH_2-)_3$ |
| $CH_3CO-$ | $CH_3$ | $C_2H_5C(CH_2-)_3$ |
| H | $n-C_4H_9$ | $C_2H_5C(CH_2-)_3$ |
| $CH_2=CHCO-$ | $n-C_4H_9$ | $-CH_2-\overset{\vert}{C}H-(CH_2)_3-CH_2-$ |

Beispiele für Verbindungen der Formel I, worin n 4 ist, sind die Verbindungen der folgenden Formeln

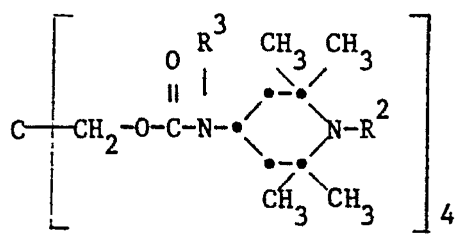

| $R^2$ | $R^3$ |
|---|---|
| H | $-CH(C_2H_5)-C_3H_7$ |
| $CH_3$ | $n-C_4H_9$ |
| $CH_3CO-$ | $-CH_2COOCH_3$ |
| H | $n-C_{12}H_{25}$ |
| $CH_3$ | $-CH_2CH_2CONH-$ 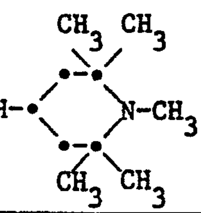 |

Die Verbindungen der Formel I können einfach hergestellt werden durch Umsetzung eines Di-, Tri- oder Tetra-Chlorcarbonates eines Polyols IV mit dem entsprechenden 4-Aminopiperidin V

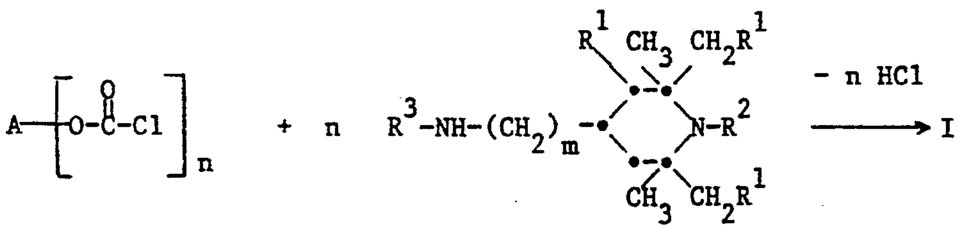


IV

V

Die Reaktion wird in einem inerten organischen Lösungsmittel ausgeführt und läuft bei Raumtemperatur praktisch quantitativ ab, wenn man den entstehenden Chlorwasserstoff durch Zusatz von n Aequivalenten einer Base neutralisiert. Als Base kann eine anorganische Base verwendet werden, wie z.B. NaOH, KOH, $NaHCO_3$, $Na_2CO_3$, $K_2CO_3$, CaO, MgO, $CaCO_3$ etc., oder eine organische Base wie ein Trialkylamin, Dialkylanilin oder Pyridin.

Besonders vorteilhaft ist die Ausführung der Reaktion in einem 2-Phasen-System. Die eine Phase besteht aus der Lösung der Komponente V in einem mit Wasser nicht mischbaren Lösungsmittel wie z.B. Toluol, Xylol, Benzol, Cyclohexan, Ligroin, Di-, Tri- oder Tetrachlormethan, 1,2-Dichlorethan, Diethylether oder Di-isopropyl-ether. Die andere Phase besteht aus der Lösung oder Suspension der Base in Wasser. Die Komponente IV wird - vorzugsweise als Lösung im verwendeten organischen Lösungsmittel - dem 2-Phasen-Gemisch unter Rühren langsam zugegeben. Die Temperatur soll dabei nicht über 80 °C steigen, vorzugsweise hält man die Temperatur bei -10 ° bis + 50 °C, insbesondere bei 0 ° bis +30 °C.

Nach der Umsetzung wird die wässrige Phase abgetrennt und die organische Phase eingedampft, wobei die Verbindungen der Formel I in hoher Ausbeute und befriedigender Reinheit erhalten werden.

Bei Ausführung der Reaktion ohne wässrige Phase kann die Base und das entstehende Basen-Hydrochlorid im verwendeten organischen Lösungsmittel löslich oder unlöslich sein. Im ersteren Fall kann das entstandene Chlorid durch Waschen mit Wasser, im zweiten Fall durch Filtration entfernt werden. Als Lösungsmittel kommen dabei auch wasserlösliche Lösungsmittel in Frage, wie z.B. Dioxan, Tetrahydrofuran, Acetonitril oder Dimethylformamid. Auch Gemische solcher polarer Lösungsmittel mit unpolaren Lösungsmitteln können verwendet werden. Bei Ausführung der Reaktion in einem Einphasensystem, ist es vorteilhaft, nach Zugabe der Komponente V das Reaktionsgemisch auf 60-150 °C zu erwärmen, um die Reaktion zu vervollständigen.

Die Verbindungen der Formel IV sind bekannte Verbindungen und lassen sich aus den entsprechenden Polyolen durch Umsetzung mit Phosgen herstellen. Die Verbindungen der Formel V, worin p null ist, sind bekannt aus den bereits erwähnten DE-OS 2 040 975, 2 349 962 und 26 21 870. Die Verbindungen der Formel V, worin p 1 oder 2 ist, können durch Hydrierung der entsprechenden Nitrile und anschliessende Einführung des Restes $R^3$ hergestellt werden, wie dies z.B. in der DE-OS 2 352 379 beschrieben ist.

Eine zweite Herstellungsmethode der Verbindungen der Formel I besteht in der Umsetzung von Alkylcarbamaten der Formel VI mit den Polyolen VII:

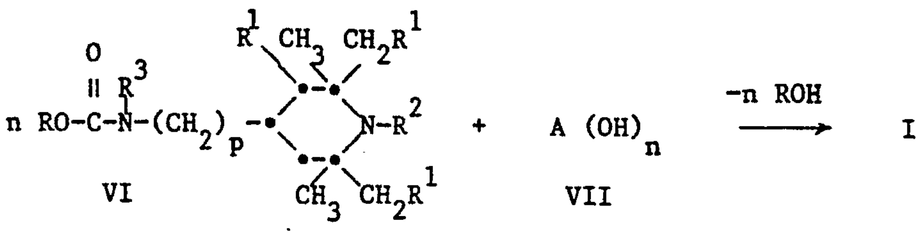

wobei R einen $C_1$-$C_4$-Alkylrest bedeutet.

Diese Reaktion wird durch Umesterungskatalysatoren, wie z.B. Alkalimetalle, Titanalkoxide oder Organozinnverbindungen, beschleunigt. Die Reaktion wird vorzugsweise ohne Lösungsmittel unter Abdestillieren des ROH bei Temperaturen von 120-200° C ausgeführt. Die dabei erhaltenen Produkte sind jedoch nicht so rein wie die nach dem ersten Verfahren gewonnenen Produkte, sodass im allgemeinen das erste Verfahren vorzuziehen ist.

Als Variante beider Verfahren kann der Rest $R^2$ auch nachträglich eingeführt werden. Hierbei wird also zuerst ein Carbamat der Formel I hergestellt, worin $R^2$ Wasserstoff ist, und in einer zweiten Reaktionsstufe dieses Wasserstoffatom gegen einen der vorhin für $R^2$ ausser Wasserstoff genannten Reste ausgetauscht. Dies kann nach den für die Substitution sekundären Amine allgemein bekannten Methoden geschehen, beispielsweise durch Umsetzung mit den entsprechenden Halogenverbindungen $R^2$ Hal in Gegenwart einer Base, die den dabei entstehenden Halogenwasserstoff bindet. Die Methylgruppe kann nach Eschweiler-Clark durch Umsetzung mit $CH_2O$/HCOOH eingeführt werden. Die Cyanomethylgruppe kann durch Umsetzung mit $CH_2O$/HCN eingeführt werden. Acylgruppen können auch durch Reaktion mit Carbonsäureanhydriden eingeführt werden. Die Einführung einer Gruppe $-CH_2CH(R^8)OR^9$ kann durch Umsetzung mit einem Oxiran

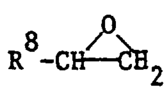

und gegebenenfalls anschliessende Verätherung oder Veresterung der resultierenden N-Hydroxyalkylverbindung nach den dafür üblichen Methoden geschehen.

Die Verbindungen der Formel I sind als Stabilisatoren für organische Polymere verwendbar, sie zeichnen sich vor allem durch hervorragende Lichtschutzwirkung aus. Beispiele für solche Polymere sind die folgenden:

1. Polymere von Mono- und Diolefinen, beispielsweise Polyethylen (das gegebenenfalls vernetzt sein kann), Polypropylen, Polyisobutylen, Polybuten-1, Polymethylpenten-1, Polyisopren oder Polybutadien sowie Polymerisate von Cycloolefinen, wie z.B. von Cyclopenten oder Norbornen.
2. Mischungen der unter 1) genannten Polymeren, z.B. Mischungen von Polypropylen mit Polyäthylen oder mit Polyisobutylen.
3. Copolymere von Mono- und Diolefinen untereinander oder mit anderen Vinylmonomeren, wie z.B. Ethylen-Propylen-Copolymere, Propylen-Buten-1-Copolymere, Propylen-Isobutylen-Copolymere, Ethylen-Buten-1-Copolymere, Propylen-Butadien-Copolymere, Isobutylen- Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen.
4. Polystyrol.
5. Copolymere von Styrol oder α-Methylstyrol mit Dienen oder Acrylderivaten, wie z.B. Styrol-Butadien,

Styrol-Acrylnitril, Styrol-Ethylmethacrylat, Styrol-Butadien-Ethylacrylat, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol, wie z.B. Styrol auf Polybutadien, Styrol und Acrylnitril auf Polybutadien, Styrol und Maleinsäureanhydrid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie z.B. als sogenannte ABS, MBS, ASA oder AES-Polymere bekannt sind.

7. Halogenhaltige Polymere, wie z.B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

8. Polymere, die sich von $\alpha,\beta$-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat-, -stearat, -benzoat, -maleat, Polyvinylbutyral, Polyallylphthalat, Polyallylmelamin.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere, wie z.B. Ethylenoxyd enthalten.

13. Polyphenylenoxyde und -sulfide.

14. Polyurethane, die sich Polyestern und Polybutadien mit endständigen Hydroxylgruppen einerseits und aliphatischen oder aromatischen Polyisocyanaten andererseits ableiten, sowie deren Vorprodukte (Polyisocyanate, Polyole, Präpolymere).

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, Polyamid 6/10, Polyamid 11, Polyamid 12, Poly-2,4,4-trimethylhexamethylen-terephthalamid, Poly-m-phenylen-isophthalamid, sowie deren Copolymere mit Polyethern, wie z.B. mit Polyethylenglykol, Polypropylenglykol oder Polytetramethylenglykol.

16. Polyharnstoffe, Polyimide und Polyamid-imide.

17. Polyester, die sich von Dicarbonsäuren und Diolen und/oder von Hydroxycarbonsäuren oder den entsprechenden Lactonen ableiten, wie Polyethylenterphthalat, Polybutylenterphthalat, Poly-1,4-dimethylolcyclohexanterephthalat, Poly-[2,2-bis(4-hydroxyphenyl)-propan]-terephthalat, Polyhydroxybenzoate, sowie Block-Polyether-ester, die sich von Polyethern mit Hydroxyendgruppen, Dialkoholen und Dicarbonsäuren ableiten.

18. Polycarbonate,

19. Polysulfone und Polyethersulfone.

20. Vernetze Polymere, die sich von Aldehyden einerseits und Phenolen, Harnstoff und Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikation.

23. Vernetzbare Acrylharze, die sich von substituierten Acrylsäureestern ableiten, wie z.B. von Epoxyacrylaten, Urethan-acrylaten oder Polyesteracrylaten.

24. Alkydharze, Polyesterharze und Acrylatharze, die mit Melaminharzen, Harnstoffharzen, Polyisocyanaten oder Epoxidharzen vernetzt sind.

25. Vernetzte Epoxidharze, die sich von Polyepoxiden ableiten.

26. Natürliche Polymere, wie Cellulose, Naturkautschuk, Gelatine, sowie deren polymeranalog chemisch abgewandelten Derivate, wie Celluloseacetate, -propionate und -butyrate, bzw. die Celluloseether, wie Methylcellulose.

Von besonderer Bedeutung ist die Stabilisierung der unter 1-3 aufgeführten Polyolefine, der unter 4-6 aufgeführten Styrolpolymerisate und der unter 14) und 15) aufgeführten Polyurethane und Polyamide.

Die erfindungsgemässen Stabilisatoren werden den Polymeren in einer Konzentration von 0,05 bis 4 Gew.-%, berechnet auf das zu stabilisierende Material, zugesetzt. Vorzugsweise werden 0,1 bis 2 Gew.-% der Verbindungen, berechnet auf das zu stabilisierende Material, in dieses eingearbeitet.

Die Einarbeitung kann vor, während oder nach der Polymerisation erfolgen, beispielsweise durch Einmischen der Verbindungen und gegebenenfalls weiterer Additive in die Schmelze nach den in der Technik üblichen Methoden, vor oder während der Formgebung. Bei Lacken erfolgt der Zusatz vorzugsweise zur Lösung des Lackes vor dessen Applikation.

Die Stabilisatoren können auch in Form eines Masterbatches, der diese Verbindungen beispielsweise in einer Konzentration von 2,5 bis 25 Gew.-% enthält, den zu stabilisierenden Polymeren zugesetzt werden.

Ausser den Verbindungen der Formel I können den Polymeren auch noch andere, bekannte Stabilisatoren zugesetzt werden. Beispiele hierfür sind die folgenden Klassen von Stabilisatoren:

1. Antioxidantien

1.1. Alkylierte Monophenole wie 2,6-Di-tert.butyl-4-methylphenol, 2-Tert.butyl-4,6-dimethylphenol, 2,6-Di-tert.butyl-4-ethylphenol, 2,6-Di-tert.butyl-4-n-butylphenol, 2,6-Di-tert.butyl-4-i-butylphenol, 2,6-Dicyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert.butyl-4-methoxymethylphenol.

1.2. Alkylierte Hydrochinone, wie 2,6-Di-tert.butyl-4-methoxyphenol, 2,5-Di-tert.butyl-hydrochinon, 2,6-Di-tert.amyl-hydrochinon, 2,6-Di-phenyl -4-octadecyloxyphenol.

1.3. Hydroxylierte Thiodiphenylether wie 2,2'-Thio-bis-(6-tert.butyl-4-methylphenol), 2,2'-Thio-bis-(4-octylphenol), 4,4'-Thio-bis-(6-tert.butyl-3-methylphenol), 4,4'-Thio-bis-(6-tert.butyl-2-methylphenol).

1.4. Alkyliden-Bisphenole wie 2,2'-Methylen-bis-(6-tert.butyl-4-methylphenol), 2,2'-Methylen-bis-(6-tert.butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert.butylphenol), 2,2'-Ethyliden- bis -(4,6-di-tert.butylphenol), 2,2'-Ethyliden-bis-(6-tert.butyl-4-isobutylphenol), 4,4'-Methylen-bis-(2,6-di-tert.butylphenol), 4,4'-Methylen-bis-(6-tert.butyl-2-methylphenol), 1,1-Bis-(5-tert.butyl-4-hydroxy-2-methylphenyl)-butan, 2,6-Di-(3-tert.butyl-5-methyl-2-hydroxybenzyl)-4-methylphenol, 1,1,3-Tris-(5-tert.butyl-4-hydroxy-2-methylphenyl)-butan, 1,1-Bis-(5-tert.butyl-4-hydroxy-2-methylphenyl)-3-n-dodecylmercaptobutan, Ethylenglycol-bis-[3,3-bis-(3'-tert.butyl-4'-hydroxyphenyl)-butyrat],Di-(3-tert.butyl-4-hydroxy-5-methylphenyl)-dicyclopentadien, Di-[2-(3'-tert.butyl-2'-hydroxy-5'-methyl-benzyl)-6-tert.butyl-4-methylphenyl]-terephthalat.

1.5. Benzylverbindungen wie 1,3,5-Tri-(3,5-di-tert.butyl-4-hydroxybenzyl)-2,4,6-trimethylbenzol, Di-(3,5-di-tert.butyl-4-hydroxybenzyl)sulfid, 3,5-Di-tert.butyl-4-hydroxybenzyl-mercaptoessigsäure-isooctylester, Bis-(4-tert.butyl-3-hydroxy-2,6-dimethylbenzyl)dithiolterephthalat, 1,3,5-Tris-(3,5-di-tert.butyl-4-hydroxybenzyl)-isocyanurat, 1,3,5-Tris-(4-tert.butyl-3-hydroxy-2,6-dimethylbenzyl)-isocyanurat, 3,5-Di-tert.butyl-4-hydroxybenzyl-phosphonsäure-dioctadecylester, 3,5-Di-tert.butyl-4-hydroxybenzyl-phosphonsäure-monoethylester-Calcium-salz.

1.6. Acylaminophenole, wie 4-Hydroxy-laurinsäureanilid, 4-Hydroxystearinsäureanilid, 2,4-Bis-octylmercapto-6-(3,5-di-tert.butyl-4-hydroxyanilino)-S-triazin.

1.7. Ester der β-(3,5-Di-tert.butyl-4-hydroxyphenyl)-propionsäure mit ein- oder mehrwertigen Alkoholen wie z.B. mit Methanol, Octadecanol, 1,6-Hexandiol, Neopentylglycol, Thiodiethylenglykol, Diethylenglycol, Triethylenglycol, Pentaerythrit, Tris-hydroxyethylisocyanurat, Di-hydroxyethyl-oxalsäurediamid.

1.8. Ester der β-(5-tert.Butyl-4-hydroxy-3-methylphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-hydroxyethylisocyanurat, Di-hydroxyethyl-oxalsäurediamid.

1.9. Amide der β-(3,5-Di-tert.butyl-4-hydroxyphenyl)-propionsäure, wie z.B. N,N'-Di-(3,5-di-tert.butyl-4-hydroxyphenylpropionyl)-hexamethylendiamin, N,N'-Di-(3,5-di-tert.butyl-4-hydroxyphenylpropionyl)-trimethylendiamin,N,N'-Di-(3,5-di-tert.butyl-4-hydroxyphenylpropionyl)-hydrazin.

2. UV-Absorber und Lichtschutzmittel

2.1. 2-(2'-Hydroxyphenyl)-benztriazole, wie z.B. das 5'-Methyl-, 3',5'-Di-tert.butyl-, 5'-Tert.butyl-, 5'-(1,1,1,3,3-Tetramethylbutyl)-,5-Chlor-3',5'-di-tert.butyl-, 5-Chlor-3'-tert.butyl-5'-methyl-,3'-sec.Butyl-5'-tert.butyl-, 4'-Octoxy-, 3',5'-Di-tert.-amyl-Derivat.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. 4-Tert.butyl-phenylsalicylat, Phenylsalicylat, Octylphenylsalicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester.

2.4. Acrylate, wie z.B. α-Cyan-β,β-diphenylacrylsäure-ethylester, bzw. -isooctylester, 2-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden wie n-Butylamin, Triäthanolamin oder N-Cyclohexyl-diethanolamin, Nickeldibutyldithiocarbamat, Nickelsalze von 4-Hydroxy-3,5-di-tert.butylbenzylphosphonsäure-monoalkylestern wie vom Methyl- oder Ethylester, Nickelkomplexe von Ketoximen wie von 2-Hydroxy-4-methyl-phenylundecylketonoxim, Nickelkomplexe des 1-Phenyl-4-lauroyl-5-hydroxypyrazols, gegebenenfalls mit zusätzlichen Liganden.

2.6. Oxalsäurediamide, wie z.B. 4,4'-Di-octyloxy-oxanilid, 2,2'-Di-octyloxy-5,5'-di-tert.butyl-oxanilid, 2,2'-Di-dodecyloxy-5,5'-di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyl-oxanilid und dessen Gemisch mit 2-Ethoxy-2'-ethyl-5,4'-di-tert.butyl-oxanilid, Gemische von ortho- und para-Methoxy- sowie von o- und p-Ethoxydisubstituierten Oxaniliden.

3. Metalldesaktivatoren, wie z.B. N,N'-Diphenyloxalsäurediamid, N-Salicylal-N'-salicyloylhydrazin, N,N'-Bis-salicyloylhydrazin, N,N'-Bis-(3,5-di-tert.butyl-4-hydroxyphenylpropionyl)-hydrazin, 3-Salicyloylamino-1,2,4-triazol, Bis-benzyliden-oxalsäuredihydrazid.

4. Phosphite und Phosphonite, wie z.B. Triphenylphosphit, Diphenylalkylphosphite, Phenyldialkylphosphite, Tri-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Di-(2,4-di-tert.butylphenyl)-pentaeryhtritdiphosphit, Tristearyl-sorbit-triphosphit, Tetrakis-(2,4-di-tert. butylphenyl)-4,4'-biphenylen-ddiphosphonit.

5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z.B. 4-tert.Butylbenzoesäure, Adipinsäure, Diphenylessigsäure.

Bei der Mitverwendung bekannter Stabilisatoren können synergistische Effekte auftreten, was besonders bei Mitverwendung von anderen Lichtschutzmitteln oder von organischen Phosphiten häufig der Fall ist. Von besonderer Bedeutung ist die Mitverwendung von Antioxydantien bei der Stabilisierung von Polyolefinen.

Weiterhin können sonstige in der Kunststoff-Technologie übliche Zusätze, wie z.B. Flammschutzmittel, Antistatica, Weichmacher, Gleitmittel, Treibmittel, Pigmente, Füllstoffe oder Verstärkungsmittel, zugesetzt werden.

Die Erfindung betrifft daher auch die durch Zusatz von 0,05 bis 4 Gew.-% mindestens einer Verbindung der Formel I stabilisierten organischen Polymeren, die gegebenenfalls noch andere bekannte und übliche Zusätze enthalten können. Die so stabilisierten Kunststoffe können in verschiedenster Form angewendet werden, z.B. als Folien, Fasern, Bändchen, Profile, oder als Bindemittel für Lacke.

Die Herstellung und Verwendung der erfindungsgemässen Verbindungen wird in den folgenden Beispielen näher beschrieben. Teile bedeuten darin Gewichtsteile und % Gewichtsprozente. Die Temperaturen sind in Celsius-Graden angegeben. Die Formel

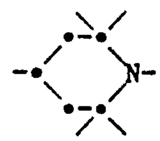

bedeutet darin einen 2,2,6,6-Tetramethylpiperidin-Rest.

Beispiel 1: In einem 4-Halskolben versehen mit Rührer, Thermometer und Tropftrichter werden die Lösungen von 50,4 g Natriumhydroxid (1,26 Mol) in 200 ml Wasser und von 255,1 g (1,2 Mol) 4-n-Butylamino-2,2,6,6-tetramethylpiperidin in 1000 ml Toluol vereinigt und auf ca. +5 °C gekühlt. Zu diesem Gemisch tropft man unter kräftigem Rühren innerhalb von ca. 6 Stunden die Lösung von 135,6 g (0,6 Mol) Butandiol-1,4-bis-chlorkohlensäureester in 250 ml Toluol, wobei die. Innentemperatur durch leichte Aussenkühlung bei 5-10 ° gehalten wird. Anschliessend wird über Nacht bei Raumtemperatur weiter gerührt. Nun werden die beiden Phasen getrennt. Die organische Phase wird dreimal mit 100 ml Wasser gewaschen, über Natriumsulfat getrocknet und das Toluol im Vakuum vollständig abdestilliert. Das in Hexan einmal umkristallisierte Biscarbamat der Struktur

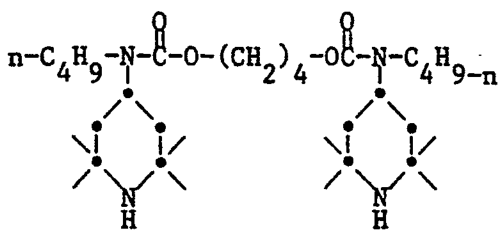

schmilzt bei 75-76 °. Das $^1$H-NMR-Spektrum steht mit der angegebenen Struktur in Einklang.

| | | | | |
|---|---|---|---|---|
| $C_{32}H_{62}N_4O_4$ | Ber: | C 67,80 | H 11,02 | N 9,88% |
| (566,87) | Gef: | C 67,91 | H 10,94 | N 9,93%. |

Analog dem Beispiel 1 werden aus den entsprechenden Aminen und Chlorkohlensäureestern die folgenden Verbindungen 1-40 hergestellt:

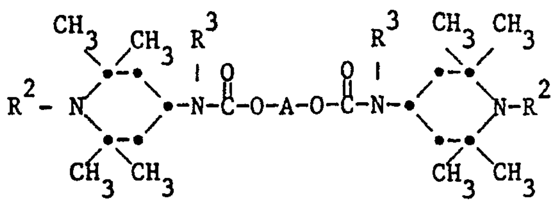

Tabelle 1

| Verbindung Nr. | $R^2$ | $R^3$ | A | Schmelzpunkt |
|---|---|---|---|---|
| 1 | H | $C_2H_5$ | $-(CH_2)_4-$ | 97-98° |
| 2 | H | $n-C_4H_9$ | $-(CH_2)_4-$ | 75-77° |
| 3 | H | $n-C_8H_{17}$ | $-(CH_2)_4-$ | 66-68° |
| 4 | H | 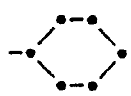 | $-(CH_2)_4-$ | 121-23° |
| 5 | Benzyl | $n-C_4H_9$ | $-(CH_2)_4-$ | 105-07° |
| 6 | H | $-CH_2CH_2OH$ | $-(CH_2)_4-$ | 153-55° |
| 7 | H | $-CH_2CH_2OOCCH_3$ | $-(CH_2)_4-$ | viskoses Oel |
| 8 | H | $n-C_4H_9$ | $-(CH_2)_2-$ | viskoses Oel |
| 9 | H | $CH_3$ | $-(CH_2)_{10}-$ | Oel |
| 10 | H | $CH_3$ | $-(CH_2)_6-$ | 120-22° |
| 11 | Allyl | $CH_3$ | $-(CH_2)_6-$ | 86-87° |
| 12 | H | $C_2H_5$ | $-(CH_2)_6-$ | 78-79° |
| 13 | H | $n-C_4H_9$ | $-(CH_2)_6-$ | 95-97° |
| 14 | $CH_2=CH-CO-$ | $n-C_4H_9$ | $-(CH_2)_6-$ | Oel |
| 15 | H | $n-C_8H_{17}$ | $-(CH_2)_6-$ | 85-86° |
| 16 | $CH_3CO-$ | $n-C_8H_{17}$ | $-(CH_2)_6-$ | 53-55° |
| 17 | H | $n-C_{12}H_{25}$ | $-(CH_2)_6-$ | 76-78° |
| 18 | $CH_3$ | $CH_3$ | $-(CH_2)_6-$ | 71-72° |
| 19 | H | 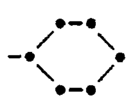 | $-(CH_2)_6-$ | 170-72° |
| 20 | H | $-CH_2COOH$ | $-(CH_2)_6-$ | ~ 230° |

Tabelle 1 (Fortsetzung)

| Verbindung Nr. | $R^2$ | $R^3$ | A | Schmelz-punkt |
|---|---|---|---|---|
| 21 | H | $-CH_2COOC_2H_5$ | $-(CH_2)_6-$ | 82-83° |
| 22 | H | $-CH_2CH_2COOC_2H_5$ | $-(CH_2)_6-$ | 96-97° |
| 23 | $CH_2{=}CH-CH_2-$ | $-CH_2COOCH_2CH{=}CH_2$ | $-(CH_2)_6-$ | viskoses Oel |
| 24 | H | $CH_3$ | $-CH_2CH_2OCH_2CH_2-$ | viskoses Oel |
| 25 | $CH_3CO-$ | $CH_3$ | $-CH_2CH_2OCH_2CH_2-$ | viskoses Oel |
| 26 | H | $n-C_4H_9$ | $-CH_2CH_2OCH_2CH_2-$ | viskoses Oel |
| 26a | H | $n-C_8H_{17}$ | $-CH_2CH_2OCH_2CH_2-$ | viskoses Oel |
| 27 | H | 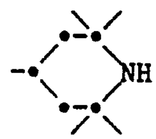 | $-(CH_2)_4-$ | 163-64° |
| 28 | H | 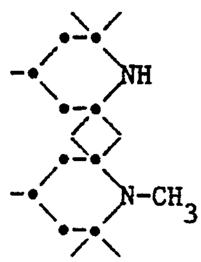 | $-(CH_2)_6-$ | 153-54° |
| 29 | $CH_3$ |  | $-(CH_2)_4-$ | 149-50° |
| 30 | H | $-CH_2COO-$ 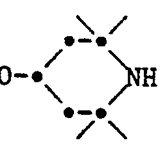 | $-(CH_2)_4-$ | 156-58° |
| 31 | H | $-CH_2COO-$ 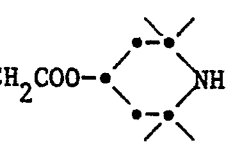 | $-(CH_2)_6-$ | 144-46° |

Tabelle 1 (Fortsetzung)

| Verbindung Nr. | $R^2$ | $R^3$ | A | Schmelzpunkt |
|---|---|---|---|---|
| 32 | Allyl | $-CH_2COO-$ 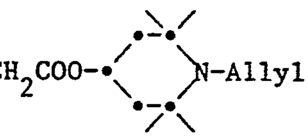 | $-(CH_2)_4-$ | Oel |
| 33 | H | $-CH_2CH_2COO-$ 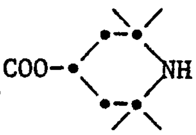 | $-(CH_2)_4-$ | 89-91° |
| 34 | H | $-CH_2CH_2COO-$ 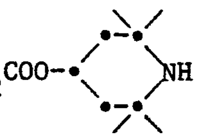 | $-(CH_2)_6-$ | 114-16° |
| 35 | H | $-CH_2CH_2COO-$ 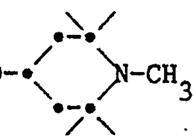 | $-(CH_2)_6-$ | 132-34° |
| 36 | $CH_3$ | $-CH_2CH_2COO-$ 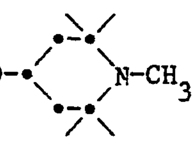 | $-(CH_2)_6-$ | 130-32° |
| 37 | H | $-CH_2CH_2CONH-$ 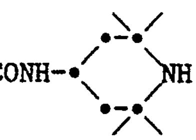 | $-(CH_2)_6-$ | ~ 80° (amorph) |
| 38 | $CH_3$ | $-CH_2CH_2CONH-$ 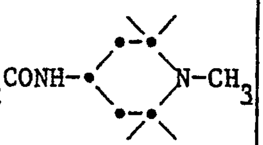 | $-(CH_2)_4-$ | ~ 90° (amorph) |
| 39 | H | $n-C_4H_9$ | $-CH_2CH_2-$ 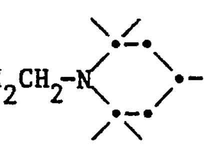 | 98-100° |

Analog werden die Verbindungen 40 und 41 hergestellt:

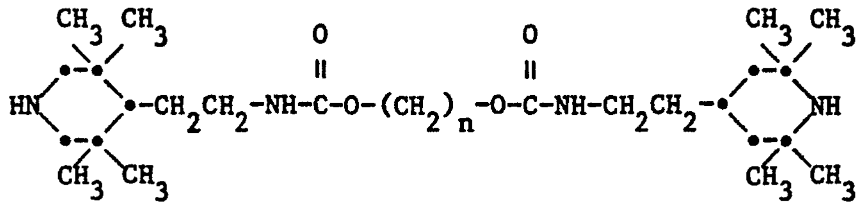

Verbindung Nr. 40 n = 4 Schmelzpunkt 96-97°
Verbindung Nr. 41 n = 6 Schmelzpunkt 78-79°.

Beispiel 2 - Stabilisierung von Polypropylen-Folie

100 Teile Polypropylenpulver (Moplen®, Fibre grade, der Firma Montedison) werden mit 0,2 Teilen β-(3,5-Di-tert.-butyl-4-hydroxyphenyl)-propionsäure-octadecylester, 0,1 Teilen Calciumstearat und 0,25 Teilen eines Stabilisators der folgenden Tabelle 2 im Brabender-Plastographen bei 200° während 10 Minuten homogenisiert. Die so erhaltene Masse wird möglichst rasch dem Kneter entnommen und in einer Kniehebelpresse zu einer 2-3 mm dicken Platte gepresst. Ein Teil des erhaltenen Rohpresslings wird ausgeschnitten und zwischen zwei Hochglanz-Hartaluminiumfolien mit einer hydraulischen Laborpresse während 6 Minuten bei 260° zu einer 0,1 mm dicken Folie gepresst, die unverzüglich in kaltem Wasser abgeschreckt wird. Aus dieser werden nun Abschnitte ausgestanzt und im Xenotest 1200 belichtet. In regelmässigen Zeitabständen werden diese Prüflinge aus dem Belichtungsapparat entnommen und in einem IR-Spektrophotometer auf ihren Carbonylgehalt geprüft. Die Zunahme der Carbonylextinktion bei 5,85 µm während der Belichtung ist ein Mass für den photooxidativen Abbau des Polymeren (s.L. Balaban et al., J. Polymer Sci, Part C; 22 (1969), 1059 - 1071) und ist erfahrungsgemäss mit einem Abfall der mechanischen Eigenschaften des Polymeren verbunden. Als Mass der Schutzwirkung gilt die Zeit bis Erreichen einer Carbonylextinktion von ca. 0,3, bei welcher die Vergleichsfolie brüchig ist.

Zur Prüfung der Flüchtigkeit und der Wasser-Extrahierbarkeit wurde a) ein Teil der Proben vor der Belichtung 7 Tage im Umluftofen auf 120° erwärmt und b) ein anderer Teil der Proben vor der Belichtung 7 Tage lang mit Wasser von 90° behandelt.

Tabelle 2 zeigt die Ergebnisse ohne und mit Vorbehandlung der Proben.

Tabelle 2

| Stabilisator Verbindung Nr. | Belichtungszeit bis zu Carbonylextinktion von 0,3 (in Stunden) | | |
|---|---|---|---|
| | ohne Vorbehandlung | 7 Tage 120° | 7 Tage Wasserextraktion bei 90° |
| - | 1100 | 290 | 310 |
| 1 | >5600 | 1320 | 690 |
| 2 | >5600 | 2920 | 2930 |
| 3 | >5750 | > 5750 | 3040 |
| 4 | >4000 | > 4000 | 3400 |
| 5 | >4200 | > 4200 | 2500 |
| 10 | 7300 | 1250 | 485 |
| 12 | >5600 | 1170 | 780 |
| 13 | 7650 | 3900 | 4200 |
| 15 | 7670 | 6700 | 3980 |
| 17 | 6700 | 6080 | 4000 |
| 19 | >5300 | > 5300 | 4150 |
| 21 | >4000 | > 4000 | 650 |
| 22 | 6150 | 5670 | 490 |
| 23 | >3000 | 2280 | 1820 |
| 27 | >5240 | >5240 | 920 |
| 28 | > 5120 | >5120 | 1190 |
| 29 | > 4200 | >4200 | 2150 |
| 31 | > 5330 | > 5330 | 680 |
| 32 | > 3000 | > 3000 | 2900 |
| 33 | >4000 | > 4000 | 810 |
| 34 | >4200 | > 4200 | 770 |
| 35 | 7940 | 5100 | 1020 |
| 36 | >6100 | >6100 | 2910 |
| 37 | 7160 | 1050 | 1230 |
| 38 | >4220 | >4220 | 1090 |
| 39 | >4200 | >4200 | > 4200 |
| 40 | >5300 | 2020 | 830 |
| 41 | >5100 | 1660 | 1600 |

Beispiel 3 - Stabilisierung eines 2-Schicht-Metalleffekt-Lackes

Aluminiumbleche von 0,5 mm Stärke werden mit einem Aluminium-pigmentierten Grundlack auf Basis Polyester/Celluloseacetobutyrat/Melaminharz beschichtet. Auf den nassen Grundlack wird ein Klarlack der folgenden Zusammensetzung aufgespritzt:

| | | |
|---|---|---|
| 58,3 Teile | Viacryl® VC 373 (Acrylharz der Fa. Vianova, Wien) | |
| 27,3 Teile | Maprenal® MF 590 (Melaminharz der Fa. Höchst AG, Frankfurt) | |
| 1,0 Teil | 1 %ige Lösung eines Silikonharzes in Xylol | |
| 4,0 Teile | Solvesso® 150 (aromatisches Lösungsmitttelgemisch) | |
| 5,4 Teile | Xylol | |
| 4,0 Teile | Ethylglykolacetat. | |

Dazu kommen 0,9 Teile des in der Tabelle 3 angegebenen Lichtschutzmittels. Dieser Klarlack hat eine Viskosität von 21 sec/DIN-Becher 4. Er wird in einer Schichtdicke von 40 µm aufgetragen und bei 130° C 39 Minuten eingebrannt.

Die Proben werden in einem UVCON - Schnellbewitterungsgerät der Fa. Atlas mit einem Zyklus von 4 h UV-Bestrahlung bei 60° und 4 h Bewitterung bei 50° 4000 h bewittert. Nach jeweils 1000 h wird der 20°-Glanz gemäss DIN 67530 gemessen. Ausserdem werden die Proben unter dem Stereomikroskop auf Rissbildung untersuchte. Die Ergebnisse sind in Tabelle 3 zusammengestellt.

Tabelle 3

| Lichtschutzmittel | 20°-Glanz nach 0 | 1000 | 2000 | 3000 | 4000 h | Rissbildung bemerkbar nach |
|---|---|---|---|---|---|---|
| keines | 97 | 47 | 9 | - | - | 2000 h |
| Verbindung Nr. 16 | 95 | 71 | 41 | 23 | - | 3000 h |
| Verbindung Nr. 25 | 96 | 78 | 61 | 22 | 18 | 4000 h |

**Patentansprüche**

1. Verbindungen der Formel

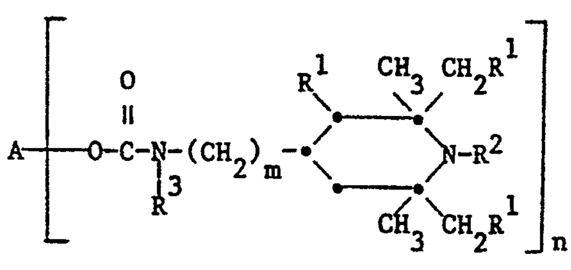

(I),

worin m null, 1 oder 2 und n 2, 3 oder 4 ist

$R^1$ Wasserstoff oder $C_1$-$C_4$-Alkyl ist,

$R^2$ Wasserstoff, $C_1$-$C_{12}$-Alkyl, $C_3$-$C_6$-Alkenyl, $C_3$-$C_5$-Alkinyl, $C_7$-$C_{12}$-Aralkyl, Glycidyl, Cyanomethyl oder eine der Gruppen $-CO-NH-R^4-$, $-CO-N(R^5)_2$, $-COOR^6$, $-CH_2COOR^6$, $-CH_2CON(R^5)_2$, $-CO-R^7$ oder $-CH_2CH(R^8)OR^9$ bedeutet,

$R^3$ Wasserstoff, $C_1$-$C_{20}$-Alkyl, durch $-OR^{10}$, $-OOC-R^{11}$, $-CN$, $-COOR^{12}$, $-N(R^{13})(R^{14})$ oder $-CON(R^{13})(R^{14})$ substituiertes $C_1$-$C_6$-Alkyl, $C_3$-$C_5$-Alkenyl, $C_5$-$C_8$-Cycloalkyl, $C_6$-$C_{12}$-Cycloalkylalkyl, $C_6$-$C_{10}$-Aryl, $C_7$-$C_{15}$-Alkylaryl, $C_7$-$C_{12}$-Aralkyl oder eine Gruppe der Formel II bedeutet,

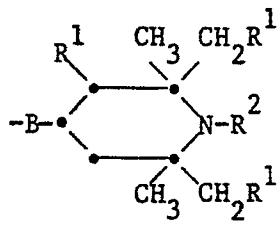

(II)

worin B eine direkte Bindung, $-CH_2CH_2-$ oder $-CH_2CH_2O-$ bedeutet,

$R^4$ $C_1$-$C_8$-Alkyl, $C_5$-$C_8$-Cycloalkyl, $C_6$-$C_{10}$-Aryl oder $C_7$-$C_{15}$-Alkylaryl bedeutet,

$R^5$ $C_1$-$C_8$-Alkyl, Cycloalkyl, Phenyl, Benzyl ist oder beide Reste $R^5$ zusammen mit dem N-Atom, an das sie gebunden sind, einen 5- bis 7-gliedrigen heterocyclischen Ring bilden,

$R^6$ $C_1$-$C_{12}$-Alkyl, $C_5$-$C_8$-Cycloalkyl, Allyl, Benzyl oder Phenyl bedeutet,

$R^7$ Wasserstoff, $C_1$-$C_{11}$-Alkyl, $C_2$-$C_5$-Alkenyl, $C_5$-$C_8$-Cycloalkyl, Phenyl oder $C_7$-$C_{12}$-Aralkyl bedeutet,

$R^8$ Wasserstoff, Methyl, Ethyl, Phenyl, Butoxymethyl, oder Phenoxymethyl bedeutet.

$R^9$ Wasserstoff, $C_1$-$C_8$ -Alkyl, $C_3$-$C_6$-Alkenyl, Benzyl oder $-CO-R^{11}$ bedeutet,

$R^{10}$ Wasserstoff, $C_1$-$C_{12}$-Alkyl oder $C_5$-$C_8$-Cycloalkyl bedeutet,

$R^{11}$ $C_1$-$C_{11}$-Alkyl, $C_2$-$C_5$-Alkenyl, $C_5$-$C_8$-Cycloalkyl, Phenyl, $C_7$-$C_{12}$-Aralkyl, durch $C_1$-$C_4$-Alkyl und/oder OH substituiertes Phenyl oder $C_7$-$C_9$-Phenylalkyl oder eine Gruppe $-NH-R^4$ bedeutet,

$R^{12}$ $C_1$-$C_{12}$-Alkyl, $C_5$-$C_8$-Cycloalkyl, Allyl, Benzyl, durch OH und $C_1$-$C_4$-Alkyl im Phenylrest substituiertes $C_7$-$C_9$-Phenylalkyl, Phenyl oder eine Gruppe der Formel II bedeutet,

$R^{13}$ $C_1$-$C_8$-Alkyl, Cyclohexyl, Benzyl, Phenyl oder einen Rest der Formel II, und

$R^{14}$ Wasserstoff, $C_1$-$C_8$-Alkyl, Cyclohexyl oder einen Rest der Formel II bedeutet oder $R^{14}$ zusammen mit $R^{13}$ und dem N-Atom einen 5-7-gliedrigen heterocyclischen Rest bildet,

A der n-wertige Rest eines Polyols ist, und wenn n 2 ist, $C_2$-$C_{16}$-Alkylen, durch ein oder mehrere -O- oder $-N(C_1$-$C_4$-Alkyl)- unterbrochenes $C_4$-$C_{20}$-Alkylen, $C_4$-$C_{10}$-Alkenylen, $C_6$-$C_8$-Cycloalkylen, $C_8$-$C_{12}$-Dialkylen-cycloalkan, $C_8$-$C_{14}$-Dialkylenaren, $C_6$-$C_{12}$-Arylen, $C_{13}$-$C_{18}$-Diphenylen-alkan oder eine Gruppe der Formel III bedeutet,

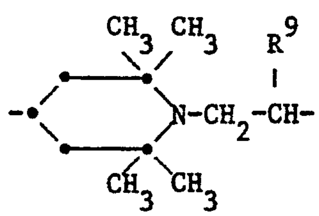

(III)

und wenn n 3 ist, A einen dreiwertigen aliphatischen oder cycloaliphatischen Kohlenwasserstoffrest mit 3-20 C-Atomen bedeutet, und wenn n 4 ist, einen vierwertigen aliphatischen Kohlenwasserstoffrest mit 4-20 C-Atomen bedeutet,
sowie Säure-Additionssalze dieser Verbindungen.

2. Verbindungen gemäss Anspruch 1 der Formel I, worin $R^1$ Wasserstoff ist.

3. Verbindungen gemäss Anspruch 1 der Formel I, worin n 2 ist.

4. Verbindungen gemäss Anspruch 1 der Formel I, worin $R^3$ nicht Wasserstoff ist.

5. Verbindungen gemäss Anspruch 1 der Formel I, worin m null und n 2 ist, $R^1$ Wasserstoff ist, $R^2$ Wasserstoff, $C_1$-$C_4$-Alkyl, Allyl, Benzyl, Cyanomethyl oder $-CO-R^7$ bedeutet, $R^3$ Wasserstoff, $C_1$-$C_{12}$-Alkyl, $C_2$-$C_3$-Hydroxyalkyl, Cyclohexyl, Phenyl, Benzyl, eine Gruppe der Formel II, $R^{11}-COOCH_2CH_2-$, $R^{12}-OOC-(CH_2)_p-$ oder $R^{13}-NH-CO-(CH_2)_p$-bedeutet, worin p 1 oder 2 ist, $R^7$ $C_1$-$C_4$-Alkyl oder Vinyl ist, $R^{11}$ $C_1$-$C_4$-Alkyl, $-NH-(C_1$-$C_4$-Alkyl), Phenyl, oder durch eine OH-Gruppe und zwei $C_1$-$C_4$-Alkylgruppen substituiertes Phenyl oder Phenylethyl bedeutet, $R^{12}$ $C_1$-$C_6$-Alkyl, Allyl, 3,5-Di-tert.butyl-4-hydroxybenzyl oder eine Gruppe der Formel II ist, $R^{13}$ $C_1$-$C_6$-Alkyl oder eine Gruppe der Formel II ist und A $C_2$-$C_{12}$-Alkylen, $C_4$-$C_6$-Mono- oder Dioxaalkylen oder eine Gruppe der Formel III bedeutet.

6. Verbindungen gemäss Anspruch 5, worin $R^3$ $C_1$-$C_{12}$-Alkyl, $C_2$-$C_3$-Hydroxyalkyl, Cyclohexyl, eine Gruppe der Formel II oder der Formel $R^{12}$-OOC-$(CH^2)_p$- ist, worin p 1 oder 2 ist und $R^{12}$ $C_1$-$C_4$-Alkyl oder eine Gruppe der Formel II ist.

7. Verbindungen gemäss Anspruch 5, worin A $C_2$-$C_6$-Alkylen oder 3-Oxa-1,5-pentylen bedeutet.

8. Verwendung von Verbindungen der Formel I gemäss Anspruch 1 als Stabilisatoren für organische Polymere.

9. Stabilisiertes organisches Polymer, enthaltend als Stabilisator 0,05 bis 4 Gewichts-% mindestens einer Verbindung der Formel I des Anspruchs 1.

## Claims

1. A compound of the formula

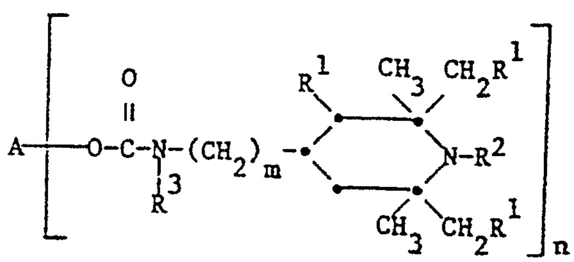

(I)

in which m is zero, 1 or 2 and n is 2, 3 or 4, $R^1$ is hydrogen or $C_1$-$C_4$alkyl, $R^2$ is hydrogen, $C_1$-$C_{12}$alkyl, $C_3$-$C_6$alkenyl, $C_3$-$C_3$alkynyl, $C_7$-$C_{12}$aralkyl, glycidyl, cyanomethyl or one of the groups -CO-NH-$R^4$, -CO-N$(R^5)_2$, -COOR$^6$, -$CH_2$COOR$^6$, -$CH_2$CON$(R^5)_2$, -CO-$R^7$ or -$CH_2$CH($R^8$)OR$^9$, $R^3$ is hydrogen, $C_1$-$C_{20}$alkyl, or $C_3$-$C_5$alkenyl, $C_5$-$C_8$cycloalkyl, $C_6$-$C_{12}$cycloalkylalkyl, $C_6$-$C_{10}$aryl, $C_7$-$C_{15}$alkylaryl, $C_7$-$C_{12}$aralkyl or $C_1$-$C_6$alkyl which is substituted by -OR$^{10}$, -OOC-$R^{11}$, -CN, -COOR$^{12}$, -N($R^{13}$)($R^{14}$) or -CON-($R^{13}$)($R^{14}$), or is a group of the formula II

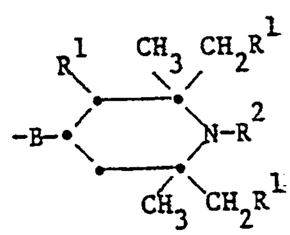

(II)

in which B is a direct bond, -$CH_2CH_2$- or -$CH_2CH_2O$-, $R^4$ is $C_1$-$C_8$alkyl, $C_5$-$C_8$cycloalkyl, $C_6$-$C_{10}$aryl or $C_7$-$C_{15}$alkylaryl, $R^5$ is $C_1$-$C_8$alkyl, cycloalkyl, phenyl or benzyl, or the two radicals $R^5$, together with the N atom to which they are attached, form a 5- to 7-membered heterocyclic ring, $R^6$ is $C_1$-$C_{12}$alkyl, $C_5$-$C_8$cycloalkyl, allyl, benzyl or phenyl, $R^7$ is hydrogen, $C_1$-$C_{11}$alkyl, $C_2$-$C_5$alkenyl, $C_5$-$C_8$cycloalkyl, phenyl or $C_7$-$C_{12}$aralkyl, $R^8$ is hydrogen, methyl, ethyl, phenyl, butoxymethyl or phenoxymethyl, $R^9$ is hydrogen, $C_1$-$C_8$alkyl, $C_3$-$C_6$alkenyl, benzyl or -CO-$R^{11}$, $R^{10}$ is hydrogen, $C_1$-$C_{12}$alkyl or $C_5$-$C_8$cycloalkyl, $R^{11}$ is $C_1$-$C_{11}$alkyl, $C_2$-$C_5$alkenyl, $C_5$-$C_8$cycloalkyl, phenyl, $C_7$-$C_{12}$aralkyl, or phenyl or $C_7$-$C_9$phenylalkyl which is substituted by $C_1$-$C_4$alkyl and/or OH, or is a group -NH-$R^4$, $R^{12}$ is $C_1$-$C_{12}$alkyl, $C_5$-$C_8$cycloalkyl, allyl, benzyl, $C_7$-$C_9$phenylalkyl which is substituted in the phenyl radical by OH and $C_1$-$C_4$alkyl, or phenyl or is a group of the formula II, $R^{13}$ is $C_1$-$C_8$alkyl, cyclohexyl, benzyl, phenyl or a radical of the formula II, and $R^{14}$ is hydrogen, $C_1$-$C_8$alkyl, cyclohexyl or a radical of the formula II, or $R^{14}$, together with $R^{13}$ and the N atom, forms a 5- to 7-membered heterocyclic radical, and A is the n-valent radical of a polyol and, if n is 2, is $C_2$-$C_{16}$alkylene or is $C_4$-$C_{10}$alkenylene, $C_6$-$C_8$cycloalkylene,

$C_8$-$C_{12}$dialkylenecycloalkane, $C_8$-$C_{14}$dialkylenearene, $C_6$-$C_{12}$arylene or $C_{13}$-$C_{18}$diphenylenealkane or $C_4$-$C_{20}$alkylene which is interrupted by one or more -O- or -N($C_1$-$C_4$alkyl)- groups, or is a group of the formula III

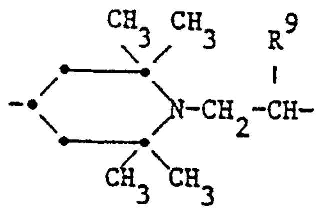

(III)

and, if n is 3, A is a trivalent aliphatic or cycloaliphatic hydrocarbon radical having 3-20 C atoms and, if n is 4, is a tetravalent aliphatic hydrocarbon radical having 4-20 C atoms, and acid addition salts of this compound.

**2.** A compound according to claim 1 of the formula I in which $R^1$ is hydrogen.

**3.** A compound according to claim 1 of the formula I in which n is 2.

**4.** A compound according to claim 1 of the formula I in which $R^3$ is not hydrogen.

**5.** A compound according to claim 1 of the formula I in which m is zero and n is 2, $R^1$ is hydrogen, $R^2$ is hydrogen, $C_1$-$C_4$alkyl, allyl, benzyl, cyanomethyl or -CO-$R^7$, and $R^3$ is hydrogen, $C_1$-$C_{12}$alkyl, $C_2$-$C_3$hydroxyalkyl, cyclohexyl, phenyl, benzyl, a group of the formula II, $R^{11}$-$COOCH_2CH_2$-, $R^{12}$-OOC-$(CH_2)_p$- or $R^{13}$-NH-CO-$(CH_2)_p$- in which p is 1 or 2, $R^7$ is $C_1$-$C_4$alkyl or vinyl, $R^{11}$ is $C_1$-$C_4$alkyl, -NH-($C_1$-$C_4$alkyl), phenyl, or phenyl or phenylethyl which is substituted by an OH group and two $C_1$-$C_4$alkyl groups, $R^{12}$ is $C_1$-$C_6$alkyl, allyl, 3,5-di-tert-butyl-4-hydroxybenzyl or a group of the formula II, $R^{13}$ is $C_1$-$C_6$alkyl or a group of the formula II and A is $C_2$-$C_{12}$alkylene, $C_4$-$C_6$monooxaalkylene, $C_4$-$C_6$dioxa-alkylene or a group of the formula III.

**6.** A compound according to claim 5, in which $R^3$ is $C_1$-$C_{12}$alkyl, $C_2$-$C_3$hydroxyalkyl, cyclohexyl or a group of the formula II or of the formula $R^{12}$-OOC-$(CH_2)_p$- in which p is 1 or 2 and $R^{12}$ is $C_1$-$C_4$alkyl or a group of the formula II.

**7.** A compound according to claim 5, in which A is $C_2$-$C_6$alkylene or 3-oxa-1,5-pentylene.

**8.** The use of a compound of the formula I according to claim 1 as a stabiliser for organic polymers.

**9.** A stabilised organic polymer containing, as the stabiliser, 0.05 to 4 % by weight of at least one compound of the formula I of claim 1.

## Revendications

**1.** Composés répondant à la formule I

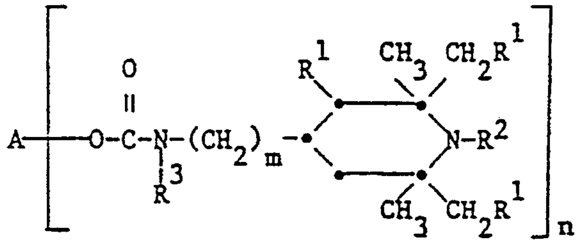

(I)

dans laquelle

m est égal à 0, à 1 ou à 2,

n est égal à 2, à 3 ou à 4,

$R^1$ représente l'hydrogène ou un alkyle en $C_1$-$C_4$,

$R^2$ représente l'hydrogène, un alkyle en $C_1$-$C_{12}$, un alcényle en $C_3$-$C_6$, un alcynyle en $C_3$-$C_5$, un aralkyle en $C_7$-$C_{12}$, un glycidyle, un cyanométhyle ou un des radicaux -CO-NH-$R^4$, -CO-N($R^5$)$_2$, -COO$R^6$, -$CH_2$COO$R^6$, -$CH_2$CON($R^5$)$_2$, -CO-$R^7$ et -$CH_2$CH($R^8$)O$R^9$,

$R^3$ représente l'hydrogène, un alkyle en $C_1$-$C_{20}$, un alkyle en $C_1$-$C_6$ porteur d'un radical -O$R^{10}$, -OOC-$R^{11}$, -CN, -COO$R^{12}$, -N($R^{13}$) ($R^{14}$) ou -CON($R^{13}$) ($R^{14}$), un alcényle en $C_3$-$C_5$, un cycloalkyle en $C_5$-$C_8$, un cycloalkyl-alkyle en $C_6$-$C_{12}$, un aryle en $C_6$-$C_{10}$, un alkylaryle en $C_7$-$C_{15}$, un aralkyle en $C_7$-$C_{12}$ ou un radical de formule II :

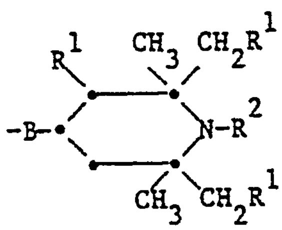

(II)

dont le symbole B représente une liaison directe ou un radical -$CH_2$-$CH_2$- ou -$CH_2$-$CH_2$O-,

$R^4$ représente un alkyle en $C_1$-$C_8$, un cycloalkyle en $C_5$-$C_8$,un aryle en $C_6$-$C_{10}$ ou un alkylaryle en $C_7$-$C_{15}$,

$R^5$ représente un alkyle en $C_1$-$C_8$, un cycloalkyle, un phényle ou un benzyle, ou les deux radicaux $R^5$ forment ensemble, et avec l'atome d'azote auquel ils sont liés, un hétérocycle contenant de 5 à 7 maillons,

$R^6$ représente un alkyle en $C_1$-$C_{12}$, un cycloalkyle en $C_5$-$C_8$, un allyle, un benzyle ou un phényle,

$R^7$ représente l'hydrogène, un alkyle en $C_1$-$C_{11}$, un alcényle en $C_2$-$C_5$, un cycloalkyle en $C_5$-$C_8$, un phényle ou un aralkyle en $C_7$-$C_{12}$,

$R^8$ représente l'hydrogène, un méthyle, un éthyle, un phényle, un butoxyméthyle ou un phénoxyméthyle,

$R^9$ représente l'hydrogène, un alkyle en $C_1$-$C_8$, un alcényle en $C_3$-$C_6$, un benzyle ou un radical -CO-$R^{11}$,

$R^{10}$ représente l'hydrogène, un alkyle en $C_1$-$C_{12}$ ou un cycloalkyle en $C_5$-$C_8$,

$R^{11}$ représente un alkyle en $C_1$-$C_{11}$, un alcényle en $C_2$-$C_5$, un cycloalkyle en $C_5$-$C_8$, un phényle, un aralkyle en $C_7$-$C_{12}$, un radical phényle ou phénylalkyle en $C_7$-$C_9$ porteur d'un alkyle en $C_1$-$C_4$ ou d'un OH, ou un radical -NH-$R^4$,

$R^{12}$ représente un alkyle en $C_1$-$C_{12}$, un cycloalkyle en $C_5$-$C_8$, un allyle, un benzyle, un phénylalkyle en $C_7$-$C_9$ dont le radical phényle porte un OH et un alkyle en $C_1$-$C_4$, un phényle ou un radical de formule II,

$R^{13}$ représente un alkyle en $C_1$-$C_8$, un cyclohexyle, un benzyle, un phényle ou un radical de formule II,

$R^{14}$ représente l'hydrogène, un alkyle en $C_1$-$C_8$, un cyclohexyle ou un radical de formule II, ou encore R14 et R13 forment ensemble, et avec l'atome d'azote, un hétérocycle contenant de 5 à 7 maillons, et

A représente le radical de valence n d'un polyol, plus précisément :

- dans le cas où n est égal à 2, un alkylène en $C_2$-$C_{16}$,un alkylène en $C_4$-$C_{20}$ interrompu par un ou plusieurs radicaux -O- ou -N($C_1$-$C_4$-alkyl)-, un alcénylène en $C_4$-$C_{10}$, un cycloalkylène en $C_6$-$C_8$, un cycloalcanedialkylène en $C_8$-$C_{12}$, un arène-dialkylène en $C_8$-$C_{14}$, un arylène en $C_6$-$C_{12}$, un alcane-diphénylène en $C_{13}$-$C_{18}$ ou un radical de formule III :

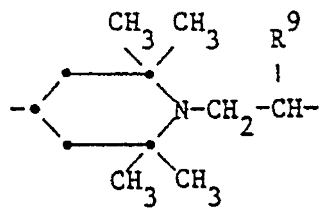

(III)

- dans le cas où n est égal à 3, un radical hydrocarboné aliphatique ou cycloaliphatique trivalent qui contient de 3 à 20 atomes de carbone, et
- dans le cas où n est égal à 4, un radical hydrocarboné aliphatique quadrivalent qui contient de 4 à 20 atomes de carbone,

ainsi que les sels d'addition que ces composés forment avec des acides.

**2.** Composés de formule I selon la revendication 1 dans lesquels $R^1$ représente l'hydrogène.

**3.** Composés de formule I selon la revendication 1 dans lesquels n est égal à 2.

**4.** Composés de formule I selon la revendication 1 dans lesquels $R^3$ ne représente pas l'hydrogène.

**5.** Composés de formule I selon la revendication 1 dans lesquels m est égal à 0, n est égal à 2, $R^1$ représente l'hydrogène, $R^2$ représente l'hydrogène, un alkyle en $C_1$-$C_4$, un allyle, un benzyle, un cyanométhyle ou -CO-$R^7$, $R^3$ représente l'hydrogène, un alkyle en $C_1$-$C_{12}$, un hydroxyalkyle en $C_2$-$C_3$, un cyclohexyle, un phényle, un benzyle, un radical de formule II, ou un radical $R^{11}$-$COOCH_2CH_2$-, $R^{12}$-OOC-$(CH_2)_p$- ou $R^{13}$-NH-CO-$(CH_2)_p$- [p étant égal à 1 ou à 2, $R^1$ étant un alkyle en $C_1$-$C_4$ ou un vinyle, $R^{11}$ un alkyle en $C_1$-$C_4$, un radical -NH-($C_1$-$C_4$-alkyl), un phényle ou un radical phényle ou phényléthyle porteur d'un -OH et de deux alkyles en $C_1$-$C_4$, $R^{12}$ un alkyle en $C_1$-$C_6$, un allyle, un di-tert-butyl-3,5 hydroxy-4 benzyle ou un radical de formule II, et $R^{13}$ un alkyle en $C_1$-$C_6$ ou un radical de formule II], et A représente un alkylène en $C_2$-$C_{12}$, un radical mono- ou dioxa-alkylène en $C_4$-$C_6$ ou un radical de formule III.

**6.** Composés selon la revendication 5 dans lesquels $R^3$ représente un alkyle en $C_1$-$C_{12}$, un hydroxyalkyle en $C_2$-$C_3$, un cyclohexyle, un radical de formule II ou un radical de formule $R^{12}$-OOC-$(CH_2)_p$- dans lequel p est égal à 1 ou à 2 et $R^{12}$ représente un alkyle en $C_1$-$C_4$ ou un radical de formule II.

**7.** Composés selon la revendication 5 dans lesquels A représente un alkylène en $C_2$-$C_6$ ou un radical oxa-3 pentylène-1,5.

**8.** Application de composés de formule I selon la revendication 1 comme stabilisants pour des polymères organiques.

**9.** Polymères organiques stabilisés qui contiennent, comme stabilisant, de 0,05 à 4 % en poids d'au moins un composé de formule I selon la revendication 1.